# FASCICULE DE BREVET EUROPEEN

(11) **EP 0 646 413 B1**
(45) Date de publication et mention de la délivrance du brevet: **01.03.2000**
(21) Numéro de dépôt: 94402045.2
(22) Date de dépôt: 13.09.1994
(51) Int. Cl.: B01J 31/24, C07C 2/36

(54) **Nouvelle composition contenant du nickel pour la catalyse et procédé de dimérisation et d'oligomérisation des oléfines**
Nickel enthaltende Katalysatorzusammensetzung und Verfahren zum Dimerisieren und Oligomerisieren von Olefinen
Nickel containing catalytic composition and process for dimerization and oligomerization of olefins

(30) Priorité: 22.09.1993 FR 9311382
(43) Date de publication de la demande: 05.04.1995
(73) Titulaire: INSTITUT FRANCAIS DU PETROLE, 92502 Rueil-Malmaison (FR)
(72) Inventeur: Chauvin, Yves, F-78230 Le Pecq (FR); Einloft, Sandra, Rio Grande Do Sul Cpe 900040 (BR); Olivier, Hélène, F-92500 Rueil Malmaison (FR)

(56) Documents cités:
- EP-A- 0 331 117
- EP-A- 0 531 174
- FR-A- 2 220 493

## Description

L' invention concerne une nouvelle composition catalytique et un procédé pour la dimérisation, la codimérisation et l'oligomérisation des oléfines, et en particulier du propylène. Ladite composition résulte du mélange d'au moins un complexe bivalent du nickel contenant deux molécules d'une phosphine tertiaire, et d'au moins un complexe du nickel bivalent ne contenant pas de phosphine et d'au moins un halogénure d'alkylaluminium. Ces mélanges sont plus particulièrement utilisables dans les compositions liquides à caractère ionique que forment les halogénures d'ammonium quaternaires, ou/et les halogénures de phosphonium quaternaires avec les halogénures d'aluminium et/ou les halogénures d'alkylaluminium et éventuellement les hydrocarbures aromatiques.

On sait que la nature des substituants portés par l'atome de phosphore des phosphines liées au nickel exerce une influence considérable sur le mode d'enchaînement des molécules d'oléfines, et plus particulièrement du propylène, lors de leur dimérisation catalytique comme décrit par G. Wilke et al. dans Ind. Eng. Chem., 1970, 62, n°12, p34, et le brevet GB 1.410.430. Pour catalyser la dimérisation G. Wilke utilise les complexes p-allyliques du nickel contenant une seule phosphine. Ces complexes sont difficiles à préparer, relativement peu stables et très sensibles à l'eau et à l'humidité. C'est pourquoi cette invention n'a jamais reçu d'application pratique. Les phosphines ont été introduites dans le catalyseur par des voies les plus diverses. En particulier les complexes que font les sels de nickel bivalents avec deux équivalents de phosphines tertiaires ont été utilisés en association avec les halogénures d'alkylalumiunium, comme catalyseurs de dimérisation des oléfines, et particulièrement du propylène. L'intérêt de l'utilisation de tels complexes réside dans leur préparation aisée et leur stabilité à l'air d'où leur facilité de mise en oeuvre. L'inconvénient de ces composés est qu'ils utilisent deux molécules de phosphine par atome de nickel, alors qu'il a été montré qu'une seule suffit pour exercer son influence. Ceci est d'autant plus regrettable que les alkylphosphines sont des composés onéreux et que d'autre part la phosphine en excès exerce un effet négatif sur la vitesse de réaction.

Il a maintenant été trouvé que le mélange (de préférence d'un équivalent) d'au moins un complexe du nickel bivalent contenant deux molécules d'une phosphine tertiaire avec (de préférence un équivalent) d'au moins un composé du nickel ne contenant pas d'eau et de phosphine et d'au moins un halogénure d'alkylaluminium conduit à des catalyseurs dont l'activité est élevée et stable et dont la sélectivité en isomères les plus ramifiés est importante et équivalente à celle qui est obtenue avec le seul complexe contenant deux phosphines, lesdits catalyseurs ayant un prix nettement inférieur.
Plus précisément, un objet de l'invention est une composition catalytique, utilisable en particulier pour la dimérisation et l'oligomérisation des oléfines, contenant au moins un halogénure d'alkylaluminium, au moins un complexe du nickel bivalent contenant deux molécules de phosphine tertiaire et au moins un composé bivalent du nickel ne contenant pas d'eau ni de phosphine.

Un autre objet de l'invention est un procédé pour la dimérisation, la codimérisation et l'oligomérisation des oléfines, procédé dans lequel au moins une oléfine est mise en contact de la composition catalytique précédente. De façon préférée, le mélange est dissous au moins en partie dans un milieu non-aqueux à caractère ionique.

Le mélange des deux types de composés du nickel doit nécessairement être associé à un halogénure d'alkylaluminium. Ce mélange peut être utilisé dans des mises en oeuvre classiques de la réaction, c'est-à-dire sans solvant ou en présence d'un hydrocarbure halogèné ou non halogèné. Il peut être utilisé en solution dans des compositions liquides non-aqueuses à caractère ionique, appelées "sels fondus", par exemple celles résultant de la mise en contact d'halogénures d'ammonium quaternaires et/ou d'halogénures de phosphonium quaternaires avec des composés d'aluminium choisis dans le groupe formé par les halogénures d'aluminium et les halogénures d'alkylaluminium, par exemple des dihalogénures d'alcoyle aluminium et éventuellement en outre un trihalogénure d'aluminium tels que décrites dans le brevet US 5.104.840.

Les complexes du nickel contenant deux molécules de phosphine selon l'invention ont pour formule générale NiX₂,2(phosphine tertiaire) dans laquelle X peut être l'anion chlorure, bromure, iodure, nitrate, acétate et X₂ l'anion sulfate Les phosphines selon l'invention répondent aux formules générales PR^{l}R²R³ et R^{l}R²P-R'-PR^{l}R² dans lesquels R^{l},R² et R³ identiques ou différents, sont des radicaux alkyl, cycloalkyl, aryl ou aralkyl comportant de 1 a 10 atomes de carbone, et R' un reste bivalent aliphatique de 1 a 6 atomes de carbone.

A titre d'exemples on peut citer :
la triisopropylphosphine,
la tricyclohexylphosphine,
la tribenzylphosphine,
la dicyclohexylphenylphosphine,
la tétra cyclohexylmethylene-diphosphine,
la diisopropyltertiobutylphosphine.

A titre d'exemples de complexes du nickel comportant deux molécules de phosphines utilisables selon l'invention on peut citer les complexes NiCl₂,2P(isopropyl)₃, NiCl₂,2P(cyclohexyl)₃.
Les composés du nickel ne comportant pas de phosphine sont les sels du nickel bivalents, non solvatés ou solvatés, la présence d'eau étant exclue, de formule générale NiX₂Lₓ dans laquelle X peut être l'anion chlorure, bromure, iodure, nitrate, carboxylate et X₂ l'anion sulfate, L peut être un composé oxygèné ou azoté tel un ether, ester, l'ammoniac, une amine primaire, secondaire ou tertiaire, un hétérocycle comportant un ou plusieurs hétéroatomes identiques ou différents, et x prenant la valeur 0, 2, 4.
A titre d'exemples de composés du nickel ne contenant pas de phosphine on peut citer les complexes NiCl₂; NiCl₂,2pyridine; NiCl₂,diméthoxyéthane; NiCl₂,4NH₃; l'acétate de nickel; I' octoate de nickel.

Les dérivés organiques de l'aluminium selon l'invention ont pour formule générale AIR_{X}X₃₋ₓ dans laquelle R est un radical alkyl, linéaire ou ramifié, comportant 2 a 8 atomes de carbone, X étant le chlore ou le brome et x ayant une valeur égale à 1, 2 ou 3. A titre d'exemples on peut utiliser le dichloroéthylaluminium, le sesquichlorure d'éthylaluminium, le sesquichlorure de d'isobutylaluminium, le dichloroisobutylaluminium et le chlorodiéthylaluminium. Le composé de l'aluminium peut être mis en oeuvre dans un rapport molaire aluminium:nickel compris entre 2:1 et 100:1, de préférence compris entre 5:1 et 50:1.

Le rapport (en équivalent Ni) entre le complexe de nickel bivalent contenant 2 molécules de phosphine tertiaire et le composé du nickel bivalent ne comportant pas d'eau ni de phosphine, est avantageusement compris entre 0,1 et 10, ou mieux entre 0,8 et 2. On préférera des rapports entre 0,8 et 1,2 et encore plus avantageusement égal à 1 ou proche de 1.

Les composés entrant dans la composition selon l'invention, peuvent être mélangés dans un ordre quelconque soit dans un solvant hydrocarboné ou hydrocarboné halogèné, tels le chlorobenzène ou le chlorure de méthylène, soit dans les produits de dimérisation ou d'oligomérisation des oléfines, soit de préférence dans un milieu liquide non-aqueux à caractère ionique.
Le mélange peut se faire par une simple mise en contact suivie d'une agitation jusqu'à formation d'un liquide homogène. Il se fait en absence d'air et de préférence en présence d'oléfine.

A titre d'exemple les milieux liquides non-aqueux à caractère ionique dans lesquels peut se dérouler la réaction sont constitués:
a) d'halogénures, plus particulièrement chlorures et/ou bromures, d'ammonium quaternaires et/ou phosphonium quaternaires;
b) d'halogénure d'aluminium et/ou d'halogénure d'alkylaluminium (trichlorure ou tribromure)
c) optionnellement et de préférence en présence d'hydrocarbures aromatiques simples, condensés et substitués ;
d) optionnellement d'un dérivé organique de l'aluminium .

Les halogénures d'ammonium quaternaires et les halogénures de phosphonium quaternaires utilisables répondent de préférence aux formules générales NR^{l}R²R³R⁴X et PR^{l}R²R³R⁴X, où X représente Cl ou Br, R^{l}, R², R³ et R⁴, identiques ou différents, représentant chacun l'hydrogène, un groupement alkyle, aliphatique (saturé ou insaturé) ou aromatique, comprenant 1 a 12 atomes de carbone. Les halogénures d'ammonium et/ou de phosphonium peuvent également être dérivés d'hétérocycles azotés ou phosphorés comportant 1, 2 ou 3 atomes d'azote et/ou de phosphore. A titre d'exemples on peut citer le chlorure de tétrabutylphosphonium, le chlorure de N-butylpyridinium, le bromure d'éthyl pyridinium, le chlorure de butyl-3 méthyl- 1 imidazolium, le chlorure de diéthylpyrazolium, le chlorhydrate de pyridinium, le chlorure de triméthylphénylammonium.

Les oléfines susceptibles d'être dimérisées, codimérisées ou oligomérisées par les compositions catalytiques selon l'invention comportent généralement 2 à 10 atomes de carbone. Ce sont de préférence des oléfines-alpha telles que l'éthylène, le propylène, les n-butènes et les n-pentènes. Les oléfines sont seules ou en mélange, pures ou diluées par exemple par un alcane, telles qu'on les trouve dans des "coupes" issues des procédés de raffinage du pétrole, comme le craquage catalytique ou le craquage a la vapeur.

La réaction catalytique de dimérisation, de co-dimérisation ou oligomérisation des oléfines peut être conduite en système fermé, en système semi-ouvert ou en continu avec un ou plusieurs étages de réaction. La température de réaction peut être comprise entre -40 et +70 °C, de préférence entre -20 et +50 °C. La chaleur engendrée par la réaction peut être éliminée par tous les moyens connus de l'homme de l'art. La pression peut être comprise entre la pression atmosphérique, ou une pression inférieure à la pression atmosphérique, et 20 MPa, de préférence entre la pression atmosphérique et 5 MPa. Les produits de la réaction et le ou les réactifs qui n'ont pas réagi sont séparés du système catalytique. Dans le cas avantageux où le procédé est conduit dans un milieu non-aqueux à caractère ionique, ces produits de la réaction et réactifs résiduels sont séparés par simple décantation puis fractionnés.

Les exemples suivants illustrent l'invention sans en limiter la portée:

### EXEMPLE 1

Un réacteur en verre de 100 ml muni d'une sonde de mesure de température, d'un barreau aimanté pour assurer une bonne agitation et d'une double enveloppe permettant la circulation d'un liquide de réfrigération, a été purgé d'air et d'humidité, et maintenu à la pression atmosphérique de propylène à 99% de pureté. On y a introduit 22 mg (0,05 mmole) du complexe NiCl₂,2P(iPr)3 et 14mg (0.05 mmole) du composé NiCl₂,2Pyridine puis on a abaissé la température a -15 °C et injecté à l'aide d'une seringue 3,5 ml de la composition liquide constituée de 0,1 mole de chlorure de butylméthyl imidazolium, de 0,122 mole de chlorure d'aluminium sublimé, de 0,002 mole de dichloroéthylaluminium et de 0.03 mole d'isodurène.On a mis l'agitation en route et on a observé immédiatement une absorption de propylène. Quand le réacteur a été aux trois quarts plein de liquide, on a arrêté l'agitation, on a laissé se décanter le "sel fondu" et on a soutiré la plus grande partie de la phase hydrocarbonée. On a recommencé l'opération six fois. A ce moment on avait introduit au total 300 g de propylène. L'analyse des différentes fractions à montré qu'elles étaient composées à 75 % de dimères, à 22 % de trimères et à 3 % de tétramères. La composition des dimères qui était pratiquement identique dans toutes les fractions comportait 81 % de diméthyl-2,3 butènes, 2 % de n-hexènes et 17 % de méthyl-2 pentènes.

### EXEMPLE 1' comparatif.

On a opéré comme dans dans l'exemple 1 à ceci près qu'on a utilisé 0,1 mmole du complexe NiCl₂,2P(iPr)₃ et pas de composé NiCl₂,2Pyridine. Les résultats ont été identiques à ceci près qu'on n'a converti que 240 g de propylène.

### EXEMPLE 2

On a opéré comme dans l'exemple 1 à ceci près qu'au lieu de d'isodurène la composition liquide contenait du toluène . On a effectué six soutirages, ce qui correspondait à 325 g de propylène introduit. Après chaque soutirage on introduisait 0.2 ml de toluène. Les six fractions étaient constituées à 78% de dimères, 19% de trimères et 3% de tétramères. Les dimères contenaient
83% de diméthyl-2,3 butènes, de 2% de n-héxènes et de 15% de méthyl-2 pentènes.

### EXEMPLE 3

On a opéré comme dans l'exemple 1 à ceci près qu'au lieu du complexe NiCl₂,2P(iPr)₃ on a utilisé la même quantité du complexe NiCl₂,2P(Bu)₃ et qu'on a opéré à +5°C. Quand le réacteur a été aux trois quarts plein de liquide, on a arrêté l'agitation, on a laissé se décanter le "sel fondu" et on a soutiré la plus grande partie de la phase hydrocarbonée. On a recommencé l'opération sept fois. A ce moment on avait introduit au total 408 g de propylène. L'analyse des différentes fractions à montré qu'elles étaient composées à 89 % de dimères, 8 % de trimères et 3 % de tétramères. La composition des dimères qui était pratiquement identique dans toutes les fractions comportait 31 % de diméthyl-2,3 butènes, 5 % de n-hexènes et 64 % de méthyl-2 pentènes.

## Revendications

1. Composition catalytique résultant du mélange d'au moins un halogénure d'alkylaluminium, d'au moins un complexe du nickel bivalent contenant deux molécules de phophine tertiaire et d'au moins un composé bivalent du nickel ne contenant pas d'eau ni de phosphine.

2. Composition catalytique selon la revendication 1 dans laquelle le rapport molaire aluminium:nickel est compris entre 2:1 et 100:1, de préférence compris entre 5:1 et 50:1.

3. Composition catalytique selon l'une des revendications 1 ou 2, dans laquelle le complexe du nickel possédant deux molécules de phosphine a pour formule générale NiX₂,2(phosphine tertiaire) dans laquelle X peut être l'anion chlorure, bromure, iodure, nitrate, acétate et X₂ l'anion sulfate.

4. Composition catalytique selon l'une des revendications 1 à 3, dans laquelle la phosphine tertiaire est choisie dans le groupe formé par la triisopropylphosphine, la tricyclohexylphosphine, la tribenzylphosphine, la dicyclohexylphenylphosphine, la tétracyclohexylmethylène-diphosphine; la diisopropyltertiobutylphosphine.

5. Composition catalytique selon l'une des revendications 1 à 4, dans laquelle les complexes contenant la phosphine sont choisis dans le groupe formé par NiCl₂,2P(isopropyl)₃ et NiCl₂,2P(cyclohexyl)₃.

6. Composition catalytique selon l'une des revendications 1 à 5, dans laquelle les composés du nickel ne comportant pas de phosphine ont pour formule générale NiX₂Lₓ dans laquelle X est l'anion chlorure, bromure, iodure, nitrate, carboxylate et X₂ l'anion sulfate, L est un composé oxygèné ou azoté tel un ether, ester, l'ammoniac, une amine primaire, secondaire ou tertiaire, un hétérocycle comportant un ou plusieurs hétéroatomes identiques ou différents, et x prenant la valeur 0, 2, 4.

7. Composition catalytique selon l'une des revendications 1 à 6, dans laquelle les composés du nickel ne contenant pas de phosphine sont choisis dans le groupe formé par NiCl₂; NiCl₂,2pyridine; NiCl₂,diméthoxyéthane; NiCl₂,4NH₃; l'acétate de nickel, I' octoate de nickel.

8. Composition catalytique selon l'une des revendications 1 à 7, dans laquelle le composé organique de l'aluminium est choisi dans le groupe formé par le dichloroéthylaluminium, le dichloroisobutylaluminium, le chlorodiéthylaluminium, le sesquichlorure d'éthylaluminium et le sesquichlorure de diisobutylaluminium.

9. Composition catalytique selon l'une des revendications 1 à 8, dans laquelle le rapport (en équivalent Ni) entre le complexe bivalent du nickel contenant deux molécules de phosphine tertiaire et le composé bivalent du nickel ne contenant pas d'eau ni de phosphine est compris entre 0,1 et 10.

10. Composition catalytique selon l'une des revendications 1 à 9, dans laquelle le rapport (en équivalent Ni) entre le complexe bivalent du nickel contenant deux molécules de phosphine tertiaire et le composé bivalent du nickel ne contenant pas d'eau ni de phosphine est compris entre 0,8 et 2.

11. Composition catalytique selon l'une des revendications 1 à 10, dans laquelle le rapport (en équivalent Ni) entre le complexe bivalent du nickel contenant deux molécules de phosphine tertiaire et le composé bivalent du nickel ne contenant pas d'eau ni de phosphine est compris entre 0,8 et 1,2.

12. Composition catalytique selon l'une des revendications 1 à 11, dans laquelle le rapport (en équivalent Ni) entre le complexe bivalent du nickel contenant deux molécules de phosphine tertiaire et le composé bivalent du nickel ne contenant pas d'eau ni de phosphine est égal à 1.

13. Composition catalytique selon l'une des revendications 1 à 12, dans laquelle ledit mélange est dissous au moins en partie dans un milieu non-aqueux à caractère ionique.

14. Composition catalytique selon l'une des revendications 1 à 12, dans laquelle ledit mélange est dissous au moins en partie dans un milieu non-aqueux à caractère ionique comprenant au moins un halogénure d'ammonium quatenaire et/ou un halogénure de phosphonium quaternaire et au moins un composé de l'aluminium choisi dans le groupe formé par les halogénures d'alkylaluminium et les halogénures d'aluminium.

15. Composition catalytique selon l'une des revendications 1 à 14, obtenue par mélange d'au moins un complexe bivalent du nickel contenant deux molécules de phosphine tertiaire avec au moins un composé bivalent du nickel ne contenant pas d'eau ni de phosphine, puis mélangé avec au moins un halogénure d'alkylaluminium.

16. Composition catalytique selon l'une des revendications 13 à 15, contenant également un hydrocarbure aromatique.

17. Procédé pour la dimérisation et l'oligomérisation et la codimérisation des oléfines, procédé dans lequel au moins une oléfine est mise en contact avec une composition catalytique selon l'une des revendications 1 à 16.

18. Procédé selon la revendication 17, dans lequel au moins une oléfine-alpha est mise en contact de la composition catalytique.

## Patentansprüche

1. Katalytische Zusammensetzung, umfassend eine Mischung von wenigstens einem Alkyl-Aluminiumhalogenid, wenigstens einem zweiwertigen Nickelkomplex, der zwei Moleküle eines tertiären Phosphins enthält, und wenigstens eine zweiwertige Nickelverbindung, die weder Wasser noch Phosphin enthält.

2. Katalytische Zusammensetzung nach Anspruch 1, wobei das molare Verhältnis von Aluminium:Nickel im Bereich zwischen 2:1 und 100:1 liegt, vorzugsweise zwischen 5:1 und 50:1 liegt.

3. Katalytische Zusammensetzung nach Anspruch 1 oder 2, wobei der Nickelkomplex zwei Moleküle Phosphin gemäß der allgemeinen Formel NiX₂,2(tertiäres Phosphin) aufweist, bei der X ein Chlorid-, Bromid-, Jodid-, Nitrat-, Acetatanion sein kann und X₂ ein Sulfatanion sein kann.

4. Katalytische Zusammensetzung nach einem der Ansprüche 1 bis 3, wobei das tertiäre Phosphin ausgewählt ist aus der Gruppe, bestehend aus Triisopropylphosphin, Tricyclohexylphosphin, Tribenzylphosphin, Dicyclohexylphenylphosphin, Tetracyclohexylmethylen-diphosphin, Diisopropyl-t-butylphosphin.

5. Zusammensetzung nach einem der Ansprüche 1 bis 4, wobei die phosphinhaltigen Komplexe ausgewählt sind aus der Gruppe, bestehend aus NiCl₂,2P(isopropyl)₃ und NiCl₂,2P(cyclohexyl)₃.

6. Katalytische Zusammensetzung nach einem der Ansprüche 1 bis 5, wobei die Nickelverbindungen, die Phosphin nicht enthalten, die allgemeine Formel NiX₂Lₓ aufweisen, bei der X ein Chlorid-, Bromid, Jodid-, Nitrat-, Carboxylatanion ist und X₂ ein Sulfatanion ist, L eine sauerstoff- oder stickstoffhaltige Verbindung ist, in Gestalt eines Ethers, Esters, von Ammoniak, einem primären, sekundären oder tertiären Amin, einem ein oder mehrere gleiche oder verschiedene Heteroatome enthaltenden Heterozyklus, und x den Wert 0, 2, 4 hat.

7. Katalytische Zusammensetzung nach einem der Ansprüche 1 bis 6, wobei die nicht Phosphin enthaltende Nickelverbindungen ausgewählt sind aus der Gruppe, bestehend aus NiCl₂, NiCl₂,2-Pyridin, NiCl₂,Dimethoxyethan, NiCl₂,4NH₃, Nickelacetat, Nickeloctoat.

8. Katalytische Zusammensetzung nach einem der Ansprüche 1 bis 7, wobei die organische Aluminiumverbindung ausgewählt ist aus der Gruppe, bestehend aus Dichlorethylaluminium, Dichlorisobutylaluminium, Chlordiethylaluminium, Ethylaluminium-sesquichlorid und Diisobutylaluminium-sesquichlorid.

9. Katalytische Zusammensetzung nach einem der Ansprüche 1 bis 8, wobei das Verhältnis (in Nickel-Äquivalenten) zwischen dem zweiwertigen Nickelkomplex, der zwei Moleküle tertiären Phosphins enthält, und der zweiwertigen Nickelverbindung, die weder Wasser noch Phosphin enthält, zwischen 0,1 und 10 liegt.

10. Katalytische Zusammensetzung nach einem der Ansprüche 1 bis 9, wobei das Verhältnis (in Ni-Äquivalenten) zwischen dem zweiwertigen Nickelkomplex, der zwei Moleküle tertiären Phosphins enthält, und der zweiwertigen Nickelverbindung, die weder Wasser noch Phosphin enthält, zwischen 0,8 und 2 liegt.

11. Katalytische Zusammensetzung nach einem der Ansprüche 1 bis 10, wobei das Verhältnis (in Ni-Äquivalenten) zwischen dem zweiwertigen Nickelkomplex, der zwei Moleküle tertiären Phosphins enthält, und der zweiwertigen Nickelverbindung, die weder Wasser noch Phosphin enthält, zwischen 0,8 und 1,2 liegt.

12. Katalytische Zusammensetzung nach einem der Ansprüche 1 bis 11, wobei das Verhältnis (in Ni-Äquivalenten) zwischen dem zweiwertigen Nickelkomplex, der zwei Moleküle tertiären Phosphins enthält, und der zweiwertigen Nickelverbindung, die weder Wasser noch Phosphin enthält, gleich 1 ist.

13. Katalytische Zusammensetzung nach einem der Ansprüche 1 bis 12, wobei die Mischung wenigstens teilweise in einem nichtwäßrigen ionischen Lösungsmittel gelöst ist.

14. Katalytische Zusammensetzung nach einem der Ansprüche 1 bis 12, wobei die Mischung wenigstens teilweise in einem nichtwäßrigen ionischen Medium gelöst ist, das wenigstens ein quartäres Ammoniumhalogenid und/oder ein quartäres Phosphoniumhalogenid enthält und wenigstens eine Aluminiumverbindung, ausgewählt aus der Gruppe, bestehend aus Alkylaluminiumhalogeniden und Aluminiumhalogeniden.

15. Katalytische Zusammensetzung nach einem der Ansprüche 1 bis 14, erhalten durch Mischung von wenigstens einem zweiwertigen Nickelkomplex, der zwei Moleküle eines tertiären Phosphins enthält, mit wenigstens einer zweiwertigen Nikkelverbindung, die weder Wasser noch Phosphin enthält, und anschließendes Mischen mit wenigstens einem Alkylaluminiumhalogenid.

16. Katalytische Zusammensetzung nach einem der Ansprüche 13 bis 15, ebenfalls enthaltend einen aromatischen Kohlenwasserstoff.

17. Verfahren zur Dimerisierung und Oligomerisierung und Codimerisierung von Olefinen, wobei wenigstens ein Olefin in Kontakt mit einer katalytischen Zusammensetzung nach einem der Ansprüche 1 bis 16 gebracht wird.

18. Verfahren nach Anspruch 17, wobei wenigstens ein α-Olefin in Kontakt mit der katalytischen Zusammensetzung gebracht wird.

## Claims

1. Catalytic composition comprising a mixture of at least one alkylaluminium halide, at least one bivalent nickel complex which contains two molecules of tertiary phosphine and at least one bivalent nickel compound containing neither water nor phosphine.

2. Catalytic composition according to claim 1 wherein the aluminium:nickel molar ratio is between 2:1 and 100:1, preferably between 5:1 and 50:1.

3. Catalytic composition according to claim 1 or claim 2 wherein the nickel complex which contains two phosphine molecules has general formula NiX₂,2(tertiary phosphine) where X represents a chloride, bromide, iodide, nitrate or acetate anion and X₂ represents the sulphate anion.

4. Catalytic composition according to any one of claims 1 to 3 wherein the tertiary phosphine is selected from the group formed by triisopropylphosphine, tricyclohexylphosphine, tribenzylphosphine, dicyclohexylphenylphosphine, tetracyclohexylmethylenediphosphine and diisopropyltertiobutylphosphine.

5. Catalytic composition according to any one of claims 1 to 4 wherein the complex which contains phosphine is selected from the group formed by NiCl₂,2P(isopropyl)₃ and NiCl₂,2P(cyclohexyl)₃.

6. Catalytic composition according to any one of claims 1 to 5 wherein the nickel compound which does not contain phosphine has general formula NiX₂Lₓ where X represents a chloride, bromide, iodide, nitrate or carboxylate anion and X₂ represents the sulphate anion, L represents an oxygenated or nitrogenated compound such as an ether, an ester, ammonia, a primary, secondary or tertiary amine, or a heterocycle containing one or more heteroatoms which may be identical or different, and x takes the value 0, 2 or 4.

7. Catalytic composition according to any one of claims 1 to 6 wherein the nickel compound which does not contain phosphine is selected from the group formed by NiCl₂; NiCl₂,2pyridine; Nicl₂,dimethoxyethane; NiCl₂,4NH₃; nickel acetate and nickel octoate.

8. Catalytic composition according to any one of claims 1 to 7 wherein the organic aluminium compound is selected from the group formed by dichloroethylaluminium, dichloroisobutylaluminium, chlorodiethylaluminium, ethylaluminium sesquichloride and diisobutylaluminium sesquichloride.

9. Catalytic composition according to any one of claims 1 to 8 wherein the ratio (in equivalents of Ni) between the bivalent nickel complex containing two tertiary phosphine molecules and the bivalent nickel compound which contains neither water nor phosphine is between 0.1 and 10.

10. Catalytic composition according to any one of claims 1 to 9 wherein the ratio (in equivalents of Ni) between the bivalent nickel complex containing two tertiary phosphine molecules and the bivalent nickel compound which contains neither water nor phosphine is between 0.8 and 2.

11. Catalytic composition according to any one of claims 1 to 10 wherein the ratio (in equivalents of Ni) between the bivalent nickel complex containing two tertiary phosphine molecules and the bivalent nickel complex which contains neither water nor phosphine is between 0.8 and 1.2.

12. Catalytic composition according to any one of claims 1 to 11 wherein the ratio (in equivalents of Ni) between the bivalent nickel complex containing two tertiary phosphine molecules and the bivalent nickel complex which contains neither water nor phosphine is equal to 1.

13. Catalytic composition according to any one of claims 1 to 12 in which said mixture is dissolved, at least in part, in an ionic non-aqueous medium.

14. Catalytic composition according to any one of claims 1 to 12 in which said mixture is dissolved, at least in part, in an ionic non-aqueous medium comprising at least one quaternary ammonium halide and/or one quaternary phosphonium halide, and at least one aluminium compound selected from the group formed by alkylaluminium halides and aluminium halides.

15. Catalytic composition according to any one of claims 1 to 14, obtained by mixing of at least one bivalent nickel complex containing two molecules of tertiary phosphine with at least one bivalent nickel compound containing neither water nor phosphine, then mixing with at least one alkylaluminium halide.

16. Catalytic composition according to any one of claims 13 to 15 containing an aromatic hydrocarbon.

17. Process for the dimerisation, codimerisaion or oligomerisation of olefins, process wherein at least one olefin is brought into contact with the catalytic composition according to any of claims 1 to 16.

18. Process according to claim 17, wherein at least one alpha-olefin is put in contact with the catalytic composition.
